# DEMANDE DE BREVET EUROPEEN

(11) **EP 1 099 763 A1**
(43) Date de publication de la demande: **16.05.2001**
(21) Numéro de dépôt: 00403094.6
(22) Date de dépôt: 08.11.2000
(51) Int. Cl.: C12P 7/18

(54) **Procédé de production de 1,3-propanediol par voie fermentaire**

(30) Priorité: 09.11.1999 FR 9914072
(71) Demandeur: Roquette Frères, 62136 Lestrem (FR)
(72) Inventeur: Defretin, Sophie, 62400 Locon (FR); Delelis, Brigitte, 62232 Vendin les Bethune (FR); Segueilha, Laurent, 59130 Lambersart (FR)
(74) Mandataire: Boulinguiez, Didier

(57) **Abrégé**

La présente invention concerne un procédé production de 1,3 propanediol par fermentation d'un microorganisme producteur de 1,3 propanediol dans un milieu de fermentation renfermant du glucose, caractérisé par le fait que l'on conduit la fermentation sans agitation mécanique en maintenant une rétention en air supérieure ou égale à 50 %, exprimée en volume de gaz sur le volume total de la phase liquide du milieu de fermentation, et en maintenant une densité cellulaire importante et une viabilité des microorganismes à une valeur, déterminée selon un test A, supérieure ou égale à 95 %, de préférence comprise entre 95 et 99 % par le contrôle de la production de mousse dans le milieu de fermentation.

## Description

La présente invention est relative à un procédé de production de 1,3 propanediol par fermentation d'un microorganisme producteur de 1,3 propanediol dans un milieu de fermentation renfermant du glucose.

En particulier, la présente invention concerne un procédé de production de 1,3 propanediol par un microorganisme producteur de 1,3 propanediol, à partir de glucose, qui consiste à conduire la fermentation sans agitation mécanique et en maintenant, dans ledit milieu de fermentation, une rétention d'air supérieure ou égale à 50 %, exprimée en volume de gaz sur le volume total de la phase liquide du milieu de fermentation, et une haute viabilité cellulaire desdits microorganismes producteurs de 1,3 propanediol.

Plus particulièrement encore, la présente invention concerne un procédé de production de 1,3 propanediol en conduisant la fermentation dans un fermenteur pneumatique de type colonne à bulles, et préférentiellement dans un fermenteur pneumatique de type colonne à bulles sans recirculation de gaz, de manière à maintenir une rétention gazeuse, une densité cellulaire et une viabilité cellulaire élevées par le contrôle de la formation de mousse dans le milieu de fermentation.

La préparation fermentaire de 1,3 propanediol s'effectue habituellement à l'aide de microorganismes des genres *Klebsiella, Citrobacter, Clostridium, Lactobacillus, Ilyobacter, Pelobacter* ou *Enterobacter,* à partir de glycérol comme source de carbone directement assimilable par ledit microorganisme et en conditions de fermentation anaérobie.

Dans ces microorganismes, le chemin métabolique qui conduit à la production de 1,3 propanediol à partir du glycérol, repose principalement sur deux activités enzymatiques successives appartenant aux voies réductrices.

La première activité enzymatique est une glycérol déshydratase qui conduit à la conversion du glycérol en 3 hydroxypropionaldéhyde. La seconde activité enzymatique est une NADH, H+ oxydoréductase qui convertit le 3 hydroxypropionaldéhyde en 1,3 propanediol.

C'est la raison pour laquelle il est admis par les spécialistes de la production du 1,3 propanediol par voie fermentaire, que ce soit à partir de glycérol pour les microorganismes naturels des genres *Klebsiella, Citrobacter, Clostridium, Lactobacillus, Ilyobacter, Pelobacter* ou *Enterobacter,* ou que ce soit à partir de glucose ou d'autres sucres, pour des microorganismes recombinants, que ladite fermentation doit s'effectuer préférentiellement en conditions anaérobies, car les voies métaboliques de conversion du glycérol en 1,3 propanediol ne sont pas des voies oxydatives, mais des voies réductrices.

Les voies oxydatives ne sont en fait à considérer que pour équilibrer le potentiel red-ox intracellulaire, en l'occurrence ici pour régénérer le cofacteur NADH,H+, nécessaire à la conversion du 3 hydroxypropionaldéhyde en 1,3 propanediol.

Les demandes de brevet WO 96/35796 et WO 98/21341 décrivent cependant le clonage des gènes codant pour ces deux activités enzymatiques et plus particulièrement le clonage et l'expression d'au moins l'activité glycérol déshydratase dans des cellules hôtes particulières, par exemple de type E. *coli* ou *S. cerevisiae* qui sont normalement fermentées en conditions aérobies.

Cette stratégie permet alors d'assurer la production de 1,3 propanediol, à partir d'une source de carbone meilleur marché que le glycérol, c'est-à-dire principalement à partir de glucose, et ce en ne mettant en oeuvre qu'un seul microorganisme recombinant.

Pourtant, dans ces demandes de brevet, si ledit microorganisme recombinant renfermant le gène codant pour l'activité déshydratase est capable de dégrader le glucose ou d'autres sucres via la voie de conversion du glycérol en 1,3 propanediol avec de bons rendement et sélectivité, les conditions de fermentation mises en oeuvre restent anaérobies.

En effet, les conditions de fermentation décrites sont des conditions de fermentation discontinue en flacons de sérum en verre agités mécaniquement, et donc au mieux microaérobies. De plus, ces conditions de fermentation ne sont pas aisément extrapolables industriellement.

De tout ce qui précède, il résulte donc qu'il existe un besoin non satisfait de disposer d'un procédé simple à mettre en oeuvre, permettant à la fois de produire ledit 1,3 propanediol de manière efficace à partir d'une source carbonée moins coûteuse que le glycérol, i.e. du glucose, et également d'obtenir ce résultat en conditions aérobies.

La société Demanderesse a donc vaincu le préjugé technique selon lequel la fermentation d'un microorganisme producteur de 1,3 propanediol ne peut être efficacement conduite qu'en conditions anaérobies, en proposant un procédé de fermentation conduite en conditions aérobies fortes grâce à la mise en oeuvre d'une fermentation non agitée mécaniquement dans des conditions particulières, qui permet par ailleurs le maintien d'une haute viabilité cellulaire des microorganismes producteurs de 1,3 propanediol.

Au sens de l'invention, on entend par " conditions aérobies fortes ", des conditions opératoires qui conduisent à maintenir dans le milieu de fermentation une valeur de rétention d'air élevée, i.e. supérieure ou égale à 50 %, de préférence supérieure à 70 %, et plus préférentiellement encore supérieure ou égale à 100 %, ce pourcentage étant exprimé en volume de gaz sur le volume total de la phase liquide dudit milieu de fermentation.

On entend également par " fermentation non agitée mécaniquement ", une fermentation conduite dans des fermenteurs pneumatiques de type colonne à bulles avec ou sans recirculation du gaz utilisé, en opposition avec les fermenteurs qui renferment un dispositif mécanique d'agitation du milieu de fermentation.

La demande de brevet WO 91/15590 décrit bien une fermentation conduite dans un réacteur de type colonne à bulles avec recirculation du gaz, permettant la production microbiologique du 1,3 propanediol, mais il s'agit ici d'un microorganisme du genre *Clostridium,* et la fermentation est réalisée base glycérol et en conditions anaérobies.

Ces conditions anaérobies conduisent à la mise en oeuvre de gaz dépourvu d'oxygène, notamment de type azote, argon ou gaz carbonique.

Ce procédé n'est donc pas du tout adapté à la fermentation de microorganismes producteurs de 1,3 propanediol par voie fermentaire en conditions aérobies.

Soucieuse de développer un procédé qui permette de répondre mieux que ceux qui existent déjà aux contraintes de la pratique, la société Demanderesse a constaté qu'une production efficace de 1,3 propanediol à partir de glucose par des microorganismes recombinants, pouvait être réalisée en conditions aérobies fortes, dans un fermenteur de type colonne à bulles sans recirculation de gaz.

De manière surprenante et inattendue, la société Demanderesse a de plus constaté que la teneur élevée en oxygène dissous peut être maintenue dans le milieu de fermentation en contrôlant la production de mousse dans le milieu de fermentation, production de mousse corrélable à la population cellulaire.

Le procédé de production de 1,3 propanediol par fermentation d'un microorganisme producteur de 1,3 propanediol dans un milieu de fermentation renfermant du glucose conforme à l'invention est caractérisé par le fait que l'on conduit la fermentation sans agitation mécanique en maintenant une rétention gazeuse supérieure ou égale à 50 %, exprimé en volume de gaz sur le volume total de la phase liquide du milieu de fermentation, et en maintenant une densité cellulaire importante et une viabilité des microorganismes à une valeur, déterminée selon un test A, supérieure ou égale à 95 %, de préférence comprise entre 95 et 99 % par le contrôle de la production de mousse dans le milieu de fermentation.

Le microorganisme producteur de 1,3 propanediol est choisi dans le groupe des microorganismes recombinants capable de produire le 1,3 propanediol à partir de glucose.

La fermentation peut être réalisée indifféremment en conditions discontinues ou continues. Il sera avantageusement choisi une fermentation discontinue, et plus préférentiellement une fermentation discontinue alimentée répétée, communément appelée fermentation fedbatch, qui permet d'alimenter en discontinu le milieu de fermentation en l'un des substrats de la fermentation, en l'occurrence ici le glucose, comme il sera exemplifié ci-après.

La société Demanderesse a remarqué que la production de 1,3 propanediol est concomitante à la croissance cellulaire et que d'autre part, la conversion totale du glucose mis en oeuvre en produits de fermentation, i.e. d'une part du 1,3 propanediol et d'autre part du glycérol et de l'acide acétique comme coproduits de la fermentation, résulte du développement d'une densité cellulaire importante et viable.

Au sens de l'invention, on entend par "une densité cellulaire importante", une population de micro organismes au moins égale à 10¹¹ cfu/ml de milieu de fermentation.

Pour cette valeur de densité cellulaire importante, la viabilité des microorganismes doit être déterminée , selon un test A, a une valeur supérieure ou égale à 95 %, de préférence comprise entre 95 et 99 %.

Pour la mesure de la viabilité cellulaire, la société Demanderesse préconise de déterminer l'état physiologique de la population de microorganismes grâce à la technique de cytométrie de flux.

Cette technique permet l'analyse de plusieurs caractéristiques cellulaires de façon individuelle et à vitesse élevée (10.000 cellules/seconde), permettant par exemple de différencier simultanément dans une population cellulaire donnée les microorganismes viables de ceux non viables et de les dénombrer avec exactitude.

Ce dénombrement est effectué par la mesure de la fluorescence émise par les microorganismes marqués au préalable avec des fluorochromes particuliers grâce à un cytomètre de flux, comme le CHEM FLOW® Autosystem à détection laser développé par la société CHEMUNEX.

La méthode comprend trois phases. La première consiste à marquer les microorganismes viables en leur faisant assimiler un substrat non fluorescent capable de traverser leur membrane cellulaire. Ce substrat non fluorescent est alors hydrolysé par les estérases de leur cytoplasme en un composé émettant une fluorescence verte et rouge lors d'une excitation laser. Seules les cellules viables sont ainsi marquées.

La deuxième phase consiste dans le marquage des microorganismes non viables qui est effectué par un réactif spécifique de l'ADN qui émet une fluorescence rouge lorsqu'il est excité par le laser.

La troisième phase consiste finalement en l'analyse de la suspension cellulaire dans l'analyseur du cytomètre de flux CHEM FLOW® Autosystem.

Les microorganismes marqués émettent le signal fluorescent qui est analysé dans le vert et dans le rouge à l'aide de deux détecteurs très sensibles. Ainsi le nombre total de microorganismes (viables et non viables) sera déterminé dans le rouge, tandis que le nombre des microorganismes viables sera déterminé à partir du signal fluorescent vert.

Le test A consiste donc, après avoir préparé une suspension cellulaire homogène renfermant de l'ordre de 5.10⁶ cfu/ml à partir du milieu de fermentation par dilutions successives avec le réactif ChemSol B7 commercialisé par la société CHEMUNEX à réaliser les étapes suivantes.

Pour le marquage de "viabilité" :
- dans un tube de 3 ml, placer 100 µl de la suspension cellulaire homogénéisée avec 900 µl de la solution de marquage de viabilité commercialisée par la société CHEMUNEX,
- homogénéiser et incuber les tubes 10 min à 30°C,
- placer les tubes dans la glace et à l'obscurité pour arrêter la réaction.

Pour le marquage de " mortalité " :
- dans un tube de 3 ml, placer 100 µl de la suspension cellulaire homogénéisée avec 900 µl de la solution de marquage de mortalité commercialisée par la société CHEMUNEX,
- homogénéiser et incuber les tubes 10 min dans la glace et à l'obscurité.

Les mesures sont ensuite réalisées sur un ChemFlow® Autosystem 3 de la manière suivante (après calibration à l'aide d'un standard spécialement mis au point pour l'analyse de suspension de bactéries):
- mélanger 150 µl de la suspension marquée avec le marquage de viabilité et 150 µl de la suspension marquée avec le marquage de mortalité,
- ajouter 1,2 ml du réactif ChemSol B7
et effectuer la mesure sur 225 µl.

L'appareil de mesure fourni une valeur de TC (ou total count) qui correspond au nombre total des événements comptabilisés lors de l'analyse, et une valeur GC, correspondant au nombre de cellules vivantes.

Le pourcentage de viabilité sera déterminé par le rapport (GC/TC)x100.

La société Demanderesse a en outre montré que le meilleur développement du microorganisme recombinant producteur de 1,3 propanediol est corrélé au transfert d'oxygène du milieu nutritif vers la cellule, seul l'oxygène dissous étant assimilé par la cellule.

Or, il est connu en toute généralité de l'homme du métier, que pour assurer ce transfert d'oxygène, il est nécessaire de disposer de moyens d'agitation et d'aération adéquats.

Il a été établi par la société Demanderesse que pour assurer un transfert d'oxygène efficace pour ces microorganismes recombinants producteurs de 1,3 propanediol base glucose en conditions aérobies, ce dernier doit atteindre une valeur supérieure à 3 g/l/h, valeur exprimée en grammes d'oxygène par litre de milieu de fermentation et par heure.

Dans ce cas, la solution habituellement préconisée par l'homme du métier est de travailler avec un apport d'oxygène pur ou d'appliquer une contre pression d'air afin d'augmenter le taux d'oxygène dissous du milieu ou encore de conduire la fermentation avec une forte agitation mécanique.

Cependant, le coût énergétique d'un tel transfert d'oxygène est trop prohibitif, et cette technique conduit à une forte lyse cellulaire par stress mécanique.

De plus, la société Demanderesse a également établi que dans le cas d'une fermentation nécessitant une telle demande en oxygène dissous, i.e. nécessitant un transfert d'oxygène supérieure ou égal à 3 g/l/h, la faisabilité industrielle d'un fermenteur où le milieu de fermentation est agité mécaniquement n'est pas concevable, étant donné les volumes utiles de plus de 100 m³ qui peuvent être mis en oeuvre.

En conséquence et conformément à la présente invention, la fermentation est conduite sans agitation mécanique, par le moyen de fermenteurs pneumatiques de type colonne à bulles et préférentiellement d'un fermenteur pneumatique de type colonne à bulles sans recirculation d'air.

L'utilisation du fermenteur pneumatique de type colonne à bulles est conseillée en toute généralité pour obtenir, à coût énergétique équivalent, un transfert d'oxygène supérieur à celui d'un fermenteur dont le milieu de fermentation est agité mécaniquement.

Cependant, les débits d'aération devant permettre d'atteindre un transfert d'oxygène supérieur à 3 g/l/h nécessitent des vitesses linéaires de gaz supérieures à 5 cm/s, qui entraînent une lyse cellulaire importante, et donc une forte production de mousse.

De manière surprenante et inattendue, la société Demanderesse a montré que l'on peut apporter la quantité en oxygène dissous requise dans le milieu de fermentation pour permettre d'assurer une production efficace de 1,3 propanediol par lesdits microorganismes recombinants, par un contrôle particulier de la formation de mousse dans le milieu de fermentation.

Il est avantageusement choisi de maintenir une rétention élevée en air et de maintenir une viabilité cellulaire élevée des microorganismes dans le milieu de fermentation en favorisant la production de mousse dans ledit milieu de fermentation.

Il est encore choisi de maîtriser préférentiellement la rétention gazeuse dans le milieu de fermentation, par l'apport de quantité élevée en air, et de limiter l'apport en antimousse, comme il sera exemplifié ci-après, plutôt que, comme il est communément admis, de gérer la fermentation par l'élimination de la production de mousse dans le milieu de fermentation.

On favorise la production de mousse dans le milieu de fermentation en mettant en oeuvre une technique choisie dans le groupe consistant à contrôler l'apport en agents anti-mousse et/ou à ajouter un surfactant. De manière préférentielle, on choisit de contrôler l'apport en agents anti-mousse.

Mais il peut être également choisi d'utiliser des surfactants afin de favoriser la formation de mousse, en combinaison avec un débit d'air élevé.

D'autres caractéristiques et avantages de l'invention apparaîtront à la lecture de l'exemple qui suit. Il n'est toutefois donné ici qu'à titre illustratif et non limitatif.

### EXEMPLE

Un milieu de croissance constitué de 10 g/1 d'extrait de levures, 16 g/l de tryptone et de 5 g/l de NaCI, est ensemencé à 0,2 % par une E. *coli* recombinante produisant du 1,3 propanediol à partir de glucose dans les conditions telles que décrites dans la demande de brevet WO 96/35796.

Cette préculture est mise à incuber à 35°C durant 4 heures afin d'atteindre une densité cellulaire représentative d'une absorbance à 550 nm de 1 unité de DO.

Un milieu de fermentation de même composition que le milieu de préculture, mais contenant en plus 10 g/1 de glucose, est ensemencé à 10 % par ladite préculture, et introduit dans un fermenteur pneumatique de type colonne à bulles sans recirculation d'air d'un volume utile de 201.

Ce milieu est alimenté par une solution de glucose à 50 % en poids sec par ajouts successifs, afin de maintenir la concentration en glucose à une valeur au plus égale à 10 g/1.

De la même manière, comme il est recommandé dans la demande de brevet WO 96/35796, un ajout de vitamine B12 à raison de 1,2 mg/h est effectué.

L'incubation est réalisée à 35°C, le pH du milieu de fermentation étant régulé à une valeur de 6,8 par ajout de NH₄OH à 20 %.

La vitesse d'alimentation d'air est de 5,4 cm/s, pour un volume de liquide de 6 l. Le volume expansé, constitué du volume du liquide plus du volume d'air est de 12 l, ce qui conduit à une rétention gazeuse de 100 %. Le taux de transfert en oxygène résultant est établi à 3 g/l/h. La viabilité cellulaire déterminée selon le test A est alors de 97 % pour une population de 2.10¹⁰cfu/ml.

L'apport d'agents antimousse EROL 18 commercialisés par la société OUVRIE est limité à une valeur de 1 ‰.

Au terme de la fermentation (i.e. jusqu'à consommation totale du glucose introduit), 70 g/l de 1,3 propanediol sont produits de manière concomitante avec 25 g/l de glycérol et 8 g/l d'acide acétique.

Cette conduite de fermentation permet un transfert en oxygène de 3 g/l/h alors que dans des conditions classiques, il aurait été de 1,5 g/l/h avec une rétention d'oxygène de 40 %.

Le procédé est donc particulièrement adapté à la production efficace de 1,3 propanediol par ces souches recombinantes aérobies.

## Revendications

1. Procédé de production de 1,3 propanediol par fermentation d'un microorganisme producteur de 1,3 propanediol dans un milieu de fermentation renfermant du glucose, caractérisé par le fait que l'on conduit la fermentation sans agitation mécanique en maintenant une rétention en air supérieure ou égale à 50 %, exprimée en volume de gaz sur le volume total de la phase liquide du milieu de fermentation, et en maintenant une densité cellulaire importante et une viabilité des microorganismes à une valeur, déterminée selon un test A, supérieure ou égale à 95 %, de préférence comprise entre 95 et 99 % par le contrôle de la production de mousse dans le milieu de fermentation.

2. Procédé selon la revendication 1, caractérisé par le fait que la fermentation sans agitation mécanique est conduite dans un fermenteur choisi dans le groupe constitué par les fermenteurs pneumatiques de type colonne à bulles et préférentiellement dans un fermenteur pneumatique de type colonne à bulles sans recirculation d'air.

3. Procédé selon l'une ou l'autre des revendications 1 et 2, caractérisé par le fait que l'on maintient une rétention élevée en air et que l'on maintient une viabilité cellulaire élevée des microorganismes dans le milieu de fermentation en favorisant la production de mousse dans ledit milieu de fermentation.

4. Procédé selon l'une quelconque des revendications 1 à 3, caractérisé par le fait que l'on favorise la production de mousse dans le milieu de fermentation en mettant en oeuvre une technique choisie dans le groupe consistant à contrôler l'apport en agents antimousse et/ou à ajouter un surfactant, plus préférentiellement consistant à contrôler l'apport en agents antimousse.

5. Procédé selon l'une quelconque des revendications 1 à 4, caractérisé par le fait que l'on conduit la fermentation de manière à maintenir une rétention en air dans le milieu de fermentation à une valeur supérieure à 70 % et de préférence supérieure ou égale à 100 %, exprimé en volume d'air sur le volume de la phase liquide total du milieu de fermentation.
